# EUROPEAN PATENT SPECIFICATION

(11) **EP 2 167 554 B1**
(45) Date of publication and mention of the grant of the patent: **13.07.2011**
(21) Application number: 08759971.8
(22) Date of filing: 23.05.2008
(51) Int. Cl.: C08F 226/06, C08F 226/10, A61K 9/51

(54) **BIOCOMPATIBLE MICROGELS AND APPLICATIONS THEREOF**
BIOKOMPATIBLE MIKROGELE UND ANWENDUNGEN DAVON
MICROGELS BIOCOMPATIBLES ET LEURS APPLICATIONS

(30) Priority: 25.05.2007 ES 200701450; 25.05.2007 US 940109 P
(43) Date of publication of application: 31.03.2010
(73) Proprietor: Universidad del Pais Vasco, 48940 Leioa Vizcaya (ES)
(72) Inventor: FORCADA GARCÍA, Jacqueline, E-48940 Leioa - Vizcaya (ES); IMAZ MAKAZAGA, Ainara, E-48940 Leioa - Vizcaya (ES); RAMOS JULIÁN, José, E-48940 Leioa - Vizcaya (ES)
(74) Representative: ABG Patentes, S.L.
(86) International application number: PCT/EP2008/056370
(87) International publication number: WO 2008/145612

(56) References cited:
- WO-A-03/063923
- US-A- 4 482 534
- US-A- 5 252 611
- MORGRET, LESLIE D. ET AL MORGRET, LESLIE D. ET AL: "Tensile properties of poly(N-vinyl caprolactam) gels Tensile properties of poly(N-vinyl caprolactam) gels" NASA/TM , NASA/TM-2004-212992, 1-10 CODEN: NATMA4; ISSN: 0499-9320 NASA/TM , NASA/TM-2004-212992, 1-10 CODEN: NATMA4; ISSN: 0499-9320, February 2004 (2004-02), XP002490671
- BOYKO, VOLODYMYR ET AL BOYKO, VOLODYMYR ET AL: "Poly(N-vinylcaprolactam) microgels. Polymeric stabilization with poly(vinyl alcohol) Poly(N-vinylcaprolactam) microgels. Polymeric stabilization with poly(vinyl alcohol)" COLLOID AND POLYMER SCIENCE , 282(2), 127-132 CODEN: CPMSB6; ISSN: 0303-402X COLLOID AND POLYMER SCIENCE , 282(2), 127-132 CODEN: CPMSB6; ISSN: 0303-402X, 15 August 2003 (2003-08-15), XP002490672
- IMAZ, AINARA ET AL: "Synthesis and characterization of saccharide-based latex particles" JOURNAL OF POLYMER SCIENCE, PART A: POLYMER CHEMISTRY , VOLUME DATE 2006, 44(1), 443-457 CODEN: JPACEC; ISSN: 0887-624X, 2005, XP002490673 cited in the application

## Description

### Field of the Invention

The present invention relates to biocompatible microgels and applications thereof, to a process for producing them and to pharmaceutical compositions comprising said microgels. Said biocompatible microgels can be used, among other applications, to transport and dose biologically active agents.

### Background of the Invention

There is a continuous development and innovation within the pharmaceutical field, in which release systems for biologically active agents constitute a very active research field. It is known that the administration of active ingredients to the human and animal body requires suitable transport carriers, in which the active ingredient remains chemically unchanged and pharmacologically stable during its transit from the site of administration to the target where it will exert its effect. Likewise, the features of the carrier must be such that it is not only compatible with the path that it must travel, but also with the dosing site, i.e. it releases the active ingredient at the precise time when it reaches the target.

Among the possibilities proposed for releasing active ingredients or drugs, nanoparticulate systems based on hydrophilic polymers have been developed as drug release systems. A number of works have been published describing several methods for preparing hydrophilic nanoparticles based on natural macromolecules such as gelatin and albumin nanoparticles.

Hydrogels and microgels are currently being proposed as new carriers for releasing active ingredients or drugs due to the fact that both can encapsulate said active ingredients or drugs in an aqueous environment and under relatively mild conditions. Furthermore, it has been verified that microgel particles can respond to stimuli much more quickly than their macroscopic gel homologs, hydrogels, due to their small size. In addition, the size of microgel particles is a very important parameter because it controls the efficacy of the release system. Particles which are especially useful in this type of application are colloidal particles with a diameter less than 1 µm [Couvreur P. et al., Polymeric Nanoparticles and Microspheres, Guiot, P.; Couvreur, P., ed., CRC Press, Boca Raton, Fla., 27-93, 1986].

The family of polymers most used in the synthesis of microgels sensitive to the conditions of the medium is that of the temperature-sensitive poly(alkylacrylamides), more specifically poly(N-isopropylacrylamide) (PNIPAM). However, the toxicity of poly(alkylacrylamides) prevents their use in biomedical applications. In spite of this fact, many articles and patents on these types of microgels have been published during the last few years: WO 2006/102762 proposes a technique for grafting boronic acid groups in PNIPAM microgels with carboxyl groups, which are used to release insulin in a controlled manner; Nolan, C. M. et al. Biomacromolecules 2006, 7 (11), 2918-2922 also investigated the release of insulin from PNIPAM microgels using variable temperature ¹H NMR; Bradley, M. et al. Langmuir 2005, 21, 1209-1215 synthesized NIPAM microgels with acrylic acid to study the capture and release of polyethylene oxide (PEO) of different molecular weights at different pHs; and Berndt, I. et al. Angew. Chem. Int. Ed. 2006, 45, 1735-1741 prepare microgels with a PNIPAM shell and a poly(N-isopropylmethacrylamide) (PNIPMAM) core, obtaining that the shell collapses at a temperature comprised between 34°C and 44°C, although the core is still swollen.

Temperature-sensitive and biocompatible monomers include N-vinylcaprolactam (VCL) [Vihola, H. et al. Biomaterials 2005, 26, 3055-3064]. Due to its biocompatibility, its use for therapeutic purposes is being investigated in the last few years. When synthesizing the corresponding polymer, different polymerization techniques have been used, such as bulk polymerization (Lau, A. C. W.; Wu, C. Macromolecules 1999, 32, 581-584); suspension polymerization (US 2003/008993), or emulsion polymerization (Peng, S.; Wu, C. Macromolecules 2001, 34, 568-571). Furthermore, copolymerizations have been carried out with N-vinylimidazole (Lozinsky, I. A. et al. Macromolecules 2003, 36, 7308-7323), sodium acrylate (Peng, S. et al. Macromol. Symp. 2000, 159, 179-186), glycidyl methacrylate (Qiu, X. et al. J. Polym. Sci. Part A, Polym. Chem. 2006, 44, 183-191), styrene (Pich, A. et al. Colloid Polym. Sci. 2003, 281, 916-920), vinylpyrrolidone (Boyko, V. et al. Macromolecules 2005, 38 (12), 5266-5270), and with methacrylic acid (Okhapkin, I. M. et al. Macromolecules 2003, 36 (21), 8130-8138). Copolymerizations have also been carried out with poly(ethylene oxide) molecules and derivatives thereof, because it is a component increasing the biocompatibility of the polymers (Laukkanen, A. et al. Macromolecules 2000, 33, 8703-8708; Yanul, N. A. et al. Macromol. Chem. Phys. 2001, 202, 1700-1709; Verbrugghe, S. et al. Macromol. Chem. Phys. 2003, 204, 1217-1225). The fact of having studied the properties of the microgels based on this monomer in depth (Laukkanen, A. et al. Macromolecules 2004, 37, 2268-2274. Meeussen, F. et al. Polymer 2000, 41, 8597-8602. Shtanko, N. I. et al. Polym. Int. 2003, 52, 1605-1610) has enabled encapsulating different compounds inside the microgel particles. For example, gold nanoparticles (Pich, A. et al. e-Polymer 2006, 18, 1-16) and enzymes (Pich, A. et al. Macromolecules 2006, 39, 7701-7707) have been introduced. It has also been possible to encapsulate several model drug molecules such as nadolol, propranolol and cholinesterase (Vihola, H. et al. Eur. J. Pharm. Sci. 2002, 16, 69-74).

Among all the families of biocompatible microgels which can be found in the literature, all those based on polysaccharides are emphasized since most of them are biodegradable. WO 2003/082316 describes the synthesis of microgels based on dextran using a crosslinker containing a covalent bond which is easily broken in acidic medium. The material is thus degraded by hydrolysis, releasing active substances in a controlled manner. Other families of microgels based on chains of polysaccharides modified with groups which can be polymerized by free radicals (WO 2006/071110 A1; Franssen, O. et al. Int. J. Pharm. 1998, 168, 1-7; Van Tomme, S.R. et al. Biomaterials 2005, 2129-2135; Chen, F. et al. Int. J. Pharm. 2006, 307, 23-32) have also been synthesized. However, none of them are temperature-sensitive.

### Brief Description of the Drawings

Figure 1 shows the variation of the average hydrodynamic diameter (Dp) of the particles of the poly(VCL-co-BA) microgel of the invention obtained in reaction I0.5CL4 (Table 1, Example 1), as a function of the temperature of the medium.
Figure 2 shows the variation of the average hydrodynamic diameter (Dp) of the particles of the poly(VCL-co-BA-co-3-MDG) microgel of the invention obtained in reaction B (Table 2, Example 1), as a function of the temperature of the medium.
Figure 3 shows the variation of the average hydrodynamic diameter (Dp) of the particles of the poly(VCL-co-PEGDA) microgel of the invention obtained in reaction PE8 (Table 3, Example 2), as a function of the temperature of the medium.
Figure 4 shows the variation of the Dp_{T}/Dp_{55°C} swelling ratio (Dp_{T} is the hydrodynamic diameter measured at each temperature and Dp_{55°C} is the deswollen hydrodynamic diameter measured at 55°C) of the particles of the poly(VCL-co-3-MDG-co-PEGDA) microgels of the invention obtained in reactions MPE4 and MPE6 (Table 4, Example 3), as a function of the temperature of the medium.
Figure 5 shows the variation curve of the average hydrodynamic diameter (Dp) of the particles of the poly(VCL-co-3-MDG-co-PEGDA-co-AA) microgel of the invention obtained in reaction AA6 (Table 5, Example 4), as a function of the temperature of the medium.
Figure 6 shows the variation curve of the average hydrodynamic diameter (Dp) of the particles of the poly(VCL-co-3-MDG-co-PEGDA-co-AA) microgel of the invention obtained in reaction AA6 (Table 5, Example 4), as a function of the pH of the medium and at 25°C.
Figure 7 shows the absorption at pH 3 of a model molecule (calcein) into biocompatible microgels of the invention obtained in reaction I1CL4B1 (Table 1, Example 1), belonging to the poly(VCL-co-BA) family, synthesized at 70°C.
Figure 8 shows the absorption at pH 3 of a model molecule (calcein) into biocompatible microgels obtained in reaction C (Table 2, Example 1), belonging to the poly(VCL-co-BA-co-3-MDG) family, synthesized at 70°C.
Figure 9 shows the absorption of a model molecule (calcein) into biocompatible microgels obtained in reaction PE6 (Table 3, Example 2), belonging to the poly(VCL-co-PEGDA) family, synthesized at 70°C.
Figure 10 shows the absorption at pH 3 of a model molecule (calcein) into biocompatible microgels obtained in reaction MPE4 (Table 4, Example 3), belonging to the poly(VCL-co-3-MDG-co-PEGDA) family, synthesized at 70°C.
Figure 11 shows the absorption at pH 3 of a model molecule (calcein) into biocompatible microgels obtained in reaction AA6 (Table 5, Example 4), belonging to the poly(VCL-co-3-MDG-co-PEGDA-co-AA) family, synthesized at 70°C.
Figure 12 shows the cell viability in 9-day embryonic rat cortical neuron *in vitro* cultures (9 DIV) after incubation for 24 hours with a biocompatible microgel of the invention, specifically with the microgel identified as I1CL4B1 [microgel obtained in Example 1 (Table 1) belonging to the poly(VCL-co-BA) family]. The 1:100 dilution (first bar) shows the lowest cell viability in these conditions; however, at 1:300 and 1:1000 dilutions (second and third bars) the cell viability is 100%.
Figure 13 shows the cell viability in 9-day embryonic rat cortical neuron *in vitro* cultures (9 DIV) after incubation for 72 hours with a biocompatible microgel of the invention, specifically with the microgel identified as I1CL4B1 [microgel obtained in Example 1 (Table 1) belonging to the poly(VCL-co-BA) family]. The 1:100 dilution (first bar) shows the lowest cell viability in these conditions; however, at 1:300 and 1:1000 dilutions (second and third bars) the cell viability is considerably higher, although it does not reach 100%.

### Detailed Description of the Invention

In an aspect, the invention relates to a biocompatible microgel, hereinafter biocompatible microgel of the invention, comprising a polymeric network, said polymeric network comprising monomeric units interconnected with one another through a crosslinking agent, in which said polymeric network can be obtained by polymerization in a dispersed medium of a biocompatible vinyl monomer and a crosslinking agent. In a particular embodiment, said polymeric network of the biocompatible microgel of the invention further comprises a comonomer based on a carbohydrate. In another particular embodiment, said polymeric network of the biocompatible microgel of the invention further comprises a comonomer based on a carbohydrate and a (meth)acrylic comonomer.

As used herein, the term "microgel" relates to a three-dimensional nanoparticle with a diameter from 10 nm to 1,000 nm (1 µm) which swells in water and is formed from a crosslinked polymer (e.g. monomeric units interconnected with one another by means of a crosslinking agent).

Likewise, as used herein, the term "biocompatible", applied to a material, relates to a non-toxic and biologically inactive material which during its use does not release any substance in harmful concentrations and does not cause adverse reactions in living beings.

If desired, said biocompatible microgel of the invention can further contain at least one biologically active agent, giving rise to a biocompatible microgel loaded with a biologically active agent which can be used to transport, supply and/or dose biologically active agents to the site of interest. The biocompatible microgels, loaded or not with biologically active agents, provided by this invention are sensitive to temperature and/or pH changes of the medium in which they are dispersed and can swell and deswell (i.e. change size), thus responding to temperature and/or pH stimuli. The nanoparticles of biocompatible microgel loaded or not with a biologically active agent provided by this invention can be lyophilized and subsequently resuspended in an aqueous medium, maintaining their properties and sensitivities unaltered. Therefore, the microgels provided by this invention are biocompatible materials useful as carriers of biologically active agents, capable of absorbing (encapsulating/uptaking) both hydrophobic and hydrophilic biologically active agents, and suitably dosing them since they respond to temperature and/or pH stimuli of the medium in which they are dispersed.

The temperature and pH-sensitive biocompatible microgels of the present invention have a number of advantages with respect to other intelligent materials which cannot be used in dosing drugs because they do not fit with the biocompatibility condition. In these new microgels, the driving force controlling the absorption and subsequent dosing of the biologically active agent has a certain covalent character, which involves an additional advantage both in the transport of the biologically active agent and its dosing in the chosen site.

### Biocompatible vinyl monomer

According to the present invention, the "biocompatible vinyl monomer" component forming the main monomer is a lactam (cyclic amide) comprising a vinyl group (-CH=CH₂) bound to the nitrogen of the lactam. It is therefore an amphiphilic monomer due to the fact that it contains hydrophilic (amide) and hydrophobic (vinyl group and alkyl groups of the lactam ring) groups.

In a particular embodiment, said biocompatible vinyl monomer is an N-vinyl-lactam with 4-7 members in the lactam ring, of general formula 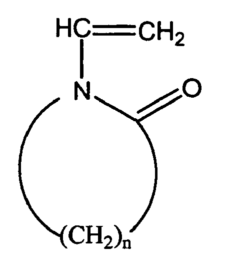 wherein n is an integer comprised between 2 and 5.

In a particular embodiment, said biocompatible vinyl monomer is selected from N-vinylcaprolactam (VCL), N-vinylpyrrolidone and mixtures thereof, preferably VCL.

VCL is partially soluble in water, its solubility in water being approximately 88 g/L at 55°C. The solubility in water of the corresponding homopolymer, poly(N-vinylcaprolactam) (PVCL), varies with temperature. The presence of the hydrophobic and hydrophilic groups makes repulsive and attractive forces coexist. The balance of these forces determines the solubility of the polymer. Certain polymers are soluble in water at low temperature, separating from the solution and forming a separate phase upon increasing the temperature above a value known as "lower critical solution temperature" (LCST). Depending on the monomers used in the polymerization reaction, the biocompatible microgels of the invention will be formed by polymers with different LCST values. The volume change that said temperature-sensitive biocompatible microgels of the invention undergo can be greater or smaller according to whether it copolymerizes with monomers more or less hydrophilic. In short, certain polymers with a suitable composition and crosslinking density can swell enormously in water at room temperature and collapse at the LCST. This phase transition is reversible.

Poly(N-vinylcaprolactam) (PVCL) is a polymer having a temperature-sensitive behavior in solution. In aqueous phase, its phase changes occur at a temperature range comprised between 30°C and 40°C, i.e. close to physiological temperature. It furthermore has an additional characteristic, its biocompatibility, which enables using it in biomedical applications. During the development of this invention it has been observed that the synthesis of said polymer is not carried out at an acidic pH for the purpose of preventing the hydrolysis of VCL.

The concentration of biocompatible vinyl monomer with respect to the total formulation (recipe) comprising the necessary components for obtaining the monomeric units interconnected with one another through a crosslinking agent forming the polymeric network of the biocompatible microgel of the invention can vary within a wide range; specifically between the concentration of biocompatible vinyl monomer which upon polymerizing does not form a macroscopic gel (maximum concentration) and the concentration of biocompatible vinyl monomer which upon polymerizing allows obtaining the desired final diameter of the biocompatible microgel of the invention (minimum concentration). In a particular embodiment, the concentration of biocompatible vinyl monomer is comprised between 1-3 weight % with respect to the total of the recipe.

### Crosslinking agent

According to the present invention, the "crosslinking agent" component is a difunctional monomer comprising at least two vinyl groups.

In a specific embodiment, said crosslinking agent comprising at least two vinyl groups is a dextran with two or more vinyl groups.

In another specific embodiment, said crosslinking agent comprising at least two vinyl groups is N,N'-methylenebisacrylamide (BA).

In another specific embodiment, said crosslinking agent is a difunctional monomer comprising at least one ethylene glycol unit in addition to at least two vinyl groups. Illustrative, non-limiting examples of said crosslinking agent include a difunctional monomer of general formula 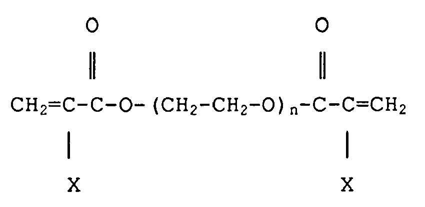 wherein
X is a hydrogen or methyl, and
n is an integer comprised between 1 and 90.

In a specific embodiment, when "n" is 1 and "X" is hydrogen, the crosslinking agent is ethylene glycol diacrylate (EGDA) and when "n" is greater than 1 and "X" is hydrogen, the crosslinking agent is poly(ethylene glycol diacrylate) (PEGDA).

In another specific embodiment, when "n" is 1 and "X" is methyl, the crosslinking agent is ethylene glycol dimethacrylate (EGDMA) and when "n" is greater than 1 and "X" is methyl, the crosslinking agent is poly(ethylene glycol dimethacrylate) (PEGDMA).

Therefore, in a particular embodiment, the crosslinking agent is a difunctional monomer selected from the group consisting of a dextran with two or more vinyl groups, BA, EGDA, PEGDA, EGDMA, PEGDMA and mixtures thereof.

Some of said crosslinking agents have the characteristic that, in addition to the oxygen atoms of the carbonyl groups, the oxygen atoms present in the ethylene glycol units can form hydrogen bonds with carboxyl groups.

The concentration of crosslinking agent, with respect to the biocompatible vinyl monomer, present in the formulation (recipe) comprising the necessary components for obtaining the monomeric units interconnected with one another through said crosslinking agent forming the polymeric network of the biocompatible microgel of the invention can vary within a wide range, depending on the desired diameter of the biocompatible microgel of the invention (at temperatures higher or lower than the LCST); nevertheless, in a particular embodiment, the concentration of crosslinking agent present in said recipe is comprised between 2 and 10 weight % with respect to the biocompatible vinyl monomer.

### Comonomer based on a carbohydrate

As has been previously mentioned, in a particular embodiment, the polymeric network of the biocompatible microgel of the invention comprises, in addition to the biocompatible vinyl monomer and the crosslinking agent, a comonomer based on a carbohydrate as an optional component.

According to the present invention, the "comonomer based on a carbohydrate" component relates to a compound comprising an acrylic or methacrylic [occasionally generically shown in this description as (meth)acrylic] group and a group derived from a glucoside. In a particular embodiment, said group derived from a glucoside comprises a glucofuran and two isopropylidene groups. This component generally increases the biocompatibility of the main monomer (biocompatible vinyl monomer) and helps in controlling the hydrophobic/hydrophilic balance of the biocompatible microgel of the invention.

In a particular and preferred embodiment, said comonomer based on a carbohydrate is selected from 3-O-methacryloyl-1,2:5,6-di-O-isopropylidene-D-glucofuranose (3-MDG), 1,2-dideoxy-4,5;6,7-di*-O-*isopropylidene-β-D-arabino-oct-1-en-3,4-diulo-4,8-pyranose, 7,8-dideoxy-1,2;3,4-di*-O-*isopropylidene-α-D-galacto-oct-7-en-1,5-pyranos-6-ulose, and mixtures thereof.

The 3-MDG compound of formula 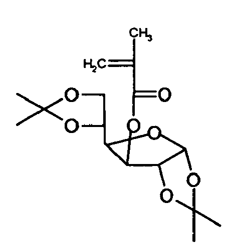 is biocompatible since it is a comonomer based on a carbohydrate and, furthermore, due to the fact that it is a more hydrophobic comonomer than VCL, it allows synthesizing biocompatible microgels of the invention with different phase transition temperatures.

The isopropylidene groups of said comonomer based on a carbohydrate can break down to give glucoside groups, each of said glucoside groups containing five hydroxyl groups in its structure.

The concentration of comonomer based on a carbohydrate, with respect to the weight amount of the biocompatible vinyl monomer, present in the formulation (recipe)comprising the necessary components for obtaining the polymeric units interconnected with one another through a crosslinking agent forming the polymeric network of the biocompatible microgel of the invention can vary within a wide range; nevertheless, in a particular embodiment, the concentration of comonomer based on a carbohydrate is comprised between 5 and 40 weight % with respect to the biocompatible vinyl monomer.

### (Meth)acrylic comonomer

As has been previously mentioned, in a particular embodiment, the polymeric network of the biocompatible microgel of the invention comprises, in addition to the biocompatible vinyl monomer and the crosslinking agent, a comonomer based on a carbohydrate and a (meth)acrylic comonomer as optional components. Illustrative, non-limiting examples of said (meth)acrylic comonomer include acrylic acid, methacrylic acid and the pH-sensitive derivatives thereof, such as N,N'-diethylaminoethyl methacrylate, aminoethyl methacrylate hydrochloride, etc. A (meth)acrylic derivative is pH-sensitive if the (meth)acrylic derivative exchanges protons when the pH varies.

This component is sensitive to pH changes of the medium and therefore confers pH sensitivity; i.e. when the pH of the medium is below the pKa of the ionizable group, said group is protonated, whereas when the pH exceeds the pKa of said group, it is deprotonated, providing a charge to the biocompatible microgel of the invention.

The concentration of (meth)acrylic comonomer with respect to the main monomer, when it is present in the formulation (recipe) comprising the necessary components for obtaining the monomeric units interconnected with one another through said crosslinking agent forming the polymeric network of the biocompatible microgel of the invention can vary within a wide range; nevertheless, in a particular embodiment, the concentration of (meth)acrylic comonomer present in said recipe is comprised between 1 and 10 weight % with respect to the biocompatible vinyl monomer.

### Obtaining the biocompatible microgel of the invention

The biocompatible microgel of the invention is obtained as a result of the polymerization of the monomeric units interconnected with one another through the crosslinking agent, generating a polymeric network. Said polymeric network can be obtained by means of a polymerization process of the monomers in a dispersed medium (biocompatible vinyl monomer) and optionally, the desired comonomers (e.g. a comonomer based on a carbohydrate and optionally, a (meth)acrylic comonomer) in the presence of said crosslinking agent. The polymerization of a monomer, as well as the copolymerization of two or more different monomers in a dispersed (heterogeneous) medium is known by persons skilled in the art. In a particular embodiment, said polymerization process is carried out by an emulsion, i.e. by means of a polymerization process in a dispersed medium by free radicals. To carry out said process, the participation of emulsifiers (surfactants) and initiators, as well as suitable buffers (regulating solutions), is required.

In a particular embodiment, the emulsion polymerization process for polymerizing said monomers and optionally, comonomers comprises the use of a suitable emulsifier, such as an anionic emulsifier, and initiator in a suitable buffer. In this type of polymerization process, water (e.g. distilled water) is generally used as a continuous reaction medium.

The anionic emulsifier confers colloidal stability to the biocompatible microgels of the invention. Although virtually any suitable anionic emulsifier could be used, in a particular embodiment, said anionic emulsifier is sodium dodecyl sulfate (SDS). The concentration of anionic emulsifier which can be used to put said process into practice can vary within a wide range; nevertheless, in a particular embodiment, the concentration of anionic emulsifier is comprised between 2 and 4 weight % with respect to the biocompatible vinyl monomer, always below the critical micelle concentration (CMC) of the emulsifier [e.g. 8.3 mM, 25°C, in the case of SDS (Gao, Y.; Au-Yeung, S. C. F.; Wu, C. Macromolecules 1999, 32, 3674-3677)], to prevent the formation of micelles.

The initiator thermally decomposes to provide free radicals which can react with the biocompatible vinyl monomer. Although virtually any suitable initiator which can break down and generate free radicals could be used, in a particular embodiment, said initiator is a peroxide, such as potassium persulfate (KPS) [K₂S₂O₈]. The concentration of initiator which can be used to put said process into practice can vary within a wide range; nevertheless, in a particular embodiment, the concentration of initiator is comprised between 0.5 and 2 weight % with respect to the biocompatible vinyl monomer.

Virtually any buffer maintaining the pH of the medium between 6-7 can be used as the buffer to be used in the polymerization reaction. In a particular embodiment, the buffer used in the polymerization reaction has been sodium bicarbonate preventing the hydrolysis of the biocompatible vinyl monomer and the decrease of the pH to 3 in the polymerization reactions initiated with potassium persulfate. The buffer will be present in the polymerization reaction medium at the suitable concentration to maintain the desired pH.

The polymerization reaction can be carried out at a temperature comprised within a wide range; nevertheless, when choosing the reaction temperature it is very important to take into account the lower critical solution temperature (LCST) of the polymeric units forming the polymeric network of the biocompatible microgel of the invention. In a particular embodiment, the polymerization reaction is carried out at a temperature greater than the LCST of the different polymeric units forming part of the biocompatible microgels of the invention. In a specific embodiment, the polymerization reaction is carried out at a temperature equal to or greater than 60°C.

Likewise, the stirring speed of the formulation (recipe) containing the necessary components for generating the biocompatible microgel of the invention by means of polymerization in a dispersed medium as has been previously mentioned can vary within a wide range; nevertheless, the stirring speed must be high enough to achieve a homogeneous mixture of the dispersion (reaction mixture) when synthesizing the biocompatible microgels of the invention. In a particular embodiment, the stirring speed is equal to or greater than 200 rpm; in another particular embodiment, the stirring speed is comprised between 200 and 400 rpm.

The polymerization reaction can be carried out in a reactor in a discontinuous or semi-continuous mode.

The technical characteristics of the biocompatible microgels of the invention will depend, among other factors, on the nature and concentration used of biocompatible vinyl monomer, crosslinking agent, comonomer based on a carbohydrate (where appropriate), (meth) acrylic comonomer (where appropriate), emulsifier, initiator and reaction temperature. However, by way of a non-limiting illustration, the values of some technical characteristics of said biocompatible microgels of the invention can be indicated in the form of a range:
- Deswollen particle diameter (55°C): 60-180 nm [average hydrodynamic particle diameter (Dp) measured by photon correlation spectroscopy (PCS)].
- Swollen particle diameter (15°C): 200-400 nm
- Lower critical solution temperature (LCST): 25-39°C
- Negative surface charge: 2.9 µC/cm² [determined by means of conductimetric titration (Ramos, J.; Forcada, J. Journal Polymer Science: Part A: Polymer Chemistry 2003, 41 (15), 2322-2334)].

The microgels of the invention are nanoparticles which, if desired, can be lyophilized and once lyophilized, if they are resuspended in an aqueous medium they maintain their properties and swelling/deswelling capacity or response to temperature and pH changes.

To analyze the sensitivity of the nanoparticles based on biocompatible microgels of the invention to temperature changes, the sizes of the final nanoparticles (Dp) were measured at different temperatures (from 10°C to 55°C, with 2.5°C interval), the LCST of the polymeric units forming them being calculated from the graphic Dp vs. T representation, T at which the inflection point of the curve is observed.

To analyze the sensitivity of the nanoparticles based on biocompatible microgels of the invention to pH changes, the sizes of the final nanoparticles (Dp) were measured at different pHs (from 2 to 10) and at 25°C.

For the biocompatible microgels of the invention to respond volumetrically to temperature and pH changes, they have to be subjected to temperature changes of the medium in which they are dispersed which vary from temperatures below the LCST to temperatures above the LCST. As regards the pH changes, the pH of the dispersion medium has to vary from pHs less than the pKₐ to values greater than the pKₐ.

### Biocompatible microgels loaded with a biologically active agent

As has been previously mentioned, the biocompatible microgels of the invention can uptake or encapsulate biologically active agents. Therefore, in a particular embodiment, the biocompatible microgel of the invention comprises a biologically active agent. Said biologically active agent can usually be inside the biocompatible microgel of the invention or nanoparticle of biocompatible microgel of the invention; nevertheless, the biologically active agent can occasionally be furthermore bound or adsorbed to the outer surface of said microgel or nanoparticle.

The biocompatible microgels loaded with biologically active agents provided by this invention, in the form of nanoparticles, are sensitive to temperature and pH changes of the medium in which they are dispersed, and can swell and deswell (i.e. change size), thus responding to temperature and pH stimuli. Nanoparticles of biocompatible microgel loaded or not with a biologically active agent provided by this invention can be lyophilized and subsequently resuspended in an aqueous medium, maintaining their properties and sensitivities unaltered.

The temperature-sensitive, and optionally pH-sensitive, biocompatible microgels of the present invention have a number of advantages with respect to other intelligent materials which cannot be used in dosing drugs because they do not fit with the biocompatibility condition. In these new microgels, the driving force controlling the absorption and subsequent dosing of the biologically active agent has a certain covalent character, which involves an additional advantage both in the transport of the biologically active agent and its dosing in the chosen site.

As used herein, the term "biologically active agent" relates to any substance which is administered to a subject, preferably a human being, with prophylactic or therapeutic purposes; i.e. any substance which can be used in the treatment, cure, prevention or diagnosis of a disease or to improve the physical and mental wellbeing of humans and animals, for example, drugs, antigens, etc.

The biocompatible microgel of the invention can incorporate one or more biologically active agents regardless of the solubility characteristics thereof.

The chemical nature of the biologically active agent can vary within a wide range, from small molecules to macromolecular molecules (peptides, polynucleotides, etc.). In a particular embodiment, said biologically active agent is a peptide or a protein. In another particular embodiment, said biologically active agent is a nucleoside, a nucleotide, an oligonucleotide, a polynucleotide or a nucleic acid (e.g. RNA or DNA). In another particular embodiment, said biologically active agent is a small (organic or inorganic) molecule; these molecules are generally obtained by semisynthetic or chemical synthesis methods or, alternatively, they are isolated from their sources. In a specific embodiment, said small (organic or inorganic) molecule has a relatively low molecular weight, generally equal to or less than 5,000, typically equal to or less than 2,500, advantageously equal to or less than 1,500. A number of therapeutic active substances (drugs) contain these characteristics and can therefore be used to put the present invention into practice.

The biocompatible microgels of the invention can be used as carriers for the administration of both hydrophilic and hydrophobic substances. Illustrative, non-limiting examples of biologically active agents (defined by therapeutic groups) which can be used and encapsulated inside said microgels include: anticarcinogenic substances, antibiotics, immunosuppressive agents, antiviral substances, enzymatic inhibitors, neurotoxins, opioids, hypnotic agents, antihistamines, tranquilizers, anticonvulsants, muscle relaxants and substances against Parkinson's disease, antispasmodic agents and muscle contractors, myotic agents and anticholinergic agents, antiglaucoma compounds, antiparasitic and/or antiprotozoal compounds, antihypertensives, analgesics, antipyretic agents and anti-inflammatory agents, local anesthetics, prostaglandins, antidepressants, antipsychotic substances, antiemetic agents, neurotransmitters, proteins, cell response modifiers and vaccines.

Illustrative, non-limiting examples of biologically active agents include actinomycin D, aldosterone, atorvastatin, carvedilol, cortisone, dexamethasone, docetaxel, erythromycin, estradiol, erlotinib, FK-506, gentamicin, hydrocortisone, indinavir, levofloxacin, methylprednisolone, methotrexate, morphine, nelfinavir, paclitaxel, pentazocin, prednisolone, prednisone, rifampicin, ritonavir, saquinavir, tacrolimus, vinblastine, vincristine, vindesine, etc., their derivatives, and mixtures thereof.

Therefore, the microgels provided by this invention are biocompatible materials useful as carriers of biologically active agents, capable of absorbing (encapsulating/uptaking) both hydrophobic and hydrophilic biologically active agents, and suitably dosing them since they respond to temperature, and optionally pH, stimuli of the medium in which they are dispersed.

The capacity to encapsulate and release biologically active agents were assayed with a model molecule, specifically calcein, a compound containing 4 carboxyl groups in its structure at room temperature and at pH 3 (below the pKₐ of the carboxyl group). When the microgel collapses, i.e. when the temperature is greater than the volume phase transition temperature (VPTT) or when the pH of the medium is greater than the pKₐ, the uptaken molecules will then be released.

The uptake or encapsulation of the biologically active agent by the biocompatible microgels of the invention can be carried out by conventional methods. In a particular embodiment, the polymerization reaction in a dispersed medium to produce the biocompatible microgel of the invention can be carried out in the presence of the biologically active agent for the purpose of encapsulating said agent therein. Alternatively, the process for obtaining the biocompatible microgel loaded with a biologically active agent provided by this invention comprises mixing a dispersion comprising a biocompatible microgel of the invention with a solution comprising said biologically active agent (or agents) to be encapsulated.

More specifically, in a particular embodiment, a suspension comprising the biocompatible microgels of the invention is mixed with a solution of the biologically active agent to be uptaken at a certain pH (e.g. pH 3, for the case of calcein); the resulting solution is stirred for a suitable time period and the microgels/nanoparticles of the invention loaded with the biologically active agent are separated by conventional methods, such as centrifugation (e.g. at 10,000-15,000 rpm and 20°C). After separating the loaded nanoparticles, the supernatant is analyzed by conventional methods, e.g. by means of spectrophotometry. Knowing the concentration of biologically active agent which was used and the concentration of biologically active agent which remained in the supernatant, the amount of biologically active agent uptaken or absorbed can be calculated.

The temperature-sensitivity of the biocompatible microgels of the invention can be used to encapsulate and release the biologically active agent. Thus, in a particular embodiment, the nanoparticles of a collapsed biocompatible microgel of the invention are placed in a solution containing the biologically active agent at a temperature less than the VPTT, whereby the particles of the microgel will swell and/or, due to interactions with the polymeric molecules, the biologically active agent molecules will penetrate through the pores of the polymeric network. If desired, the microgels of the invention loaded with the biologically active agent can be removed from the solution containing the biologically active agent by conventional methods, by centrifugation, for example. When the temperature of the medium is greater than the VPTT, the biologically active agent is released. The use of VCL as a monomer in the production of microgels allows developing families of biocompatible microgels the VPTT of which can be adjusted to body temperature for the purpose of releasing the biologically active agent in the site of interest or in the site of action.

Therefore, the biocompatible microgels of the invention can work as transport system for biologically active molecules. By way of illustration, the controlled supply of drugs is very interesting in a number of occasions, for example in the treatment of pain/diseases affecting the nervous system, e.g. in the treatment of pain caused by central nervous system (CNS) disorders, chemotherapy of intracerebral tumors and metastases, spinal chord injuries and post-trauma cerebral pain.

### Pharmaceutical compositions

In another aspect, the invention relates to a pharmaceutical composition comprising a biocompatible microgel loaded with a biologically active agent provided by this invention and a pharmaceutically acceptable excipient, carrier or adjuvant.

The biologically active agent present in the biocompatible microgel of the invention will generally be inside the biocompatible microgel of the invention; nevertheless, it could occur that part of said biologically active agent were also bound to the surface of the biocompatible microgel of the invention although the greater part of it will be inside (e.g. encapsulated) the biocompatible microgel of the invention.

Illustrative, non-limiting examples of pharmaceutical compositions include any (solid or semisolid) composition intended for its oral, buccal, sublingual, parenteral (e.g. subcutaneous, specific injection for the site of action, intramuscular, etc.), topical, ocular, intranasal, pulmonary, rectal, vaginal administration, etc.

In a particular embodiment, the pharmaceutical composition is administered orally due to its biocompatibility. The biocompatible microgels of the invention are "intelligent" materials providing a more controlled release of the biologically active agent and protecting said biologically active agents during the release, their bioavailability thus being able to be uniformly and constantly controlled. The reversibility in the swelling properties of these microgels makes these materials excellent transport carriers for both small-sized biologically active agents and for new macromolecular drugs (peptides, for example) and other therapeutic products.

Illustrative, non-limiting examples of pharmaceutical compositions include suspensions of the biocompatible microgels loaded with biologically active agents provided for their oral administration, etc. Therefore, in a particular embodiment, the pharmaceutical composition provided by this invention is administered orally.

Likewise, the pharmaceutical compositions of the invention can also be adapted for their parenteral administration in the form of, for example, suspensions or lyophilized products suitable for their reconstitution prior to their administration, in the suitable dosage form.

The pharmaceutical compositions described' herein will include the suitable pharmaceutically acceptable carriers and excipients for each formulation, which will be chosen according to the chosen pharmaceutical dosage form. A review of the different pharmaceutical dosage forms for drugs and of their preparation can be found in the book "Tratado de Farmacia Galenica", by C. Faulí i Trillo, 10th Edition, 1993, Luzán 5, S.A. de Ediciones.

The proportion of the biologically active agent incorporated in the biocompatible microgels of the invention can vary within a wide range, for example it can be up to 50 weight % with respect to the total weight of the nanoparticles. Nevertheless, the suitable proportion will depend in each case on the biologically active agent incorporated.

The invention is described below by means of several examples which do not limit, but rather illustrate the invention.

### EXAMPLE 1

### Synthesis of microgels based on N-vinylcaprolactam [poly(VCL-co-BA) and poly(VCL-co-BA-co-3-MDG) microgels]

### 1. Materials

The monomer, N-vinylcaprolactam (VCL, Aldrich); the initiator: potassium persulfate (K₂S₂O₈, Fluka); the buffer: sodium bicarbonate (NaHCO₃); the crosslinking agent: N,N'-methylenebisacrylamide (BA, Aldrich); the emulsifier: sodium dodecyl sulfate (SDS, Aldrich); and the ¹H-NMR standard, sodium acetate, were used as they were purchased. Hydroquinone (Fluka) was used as the polymerization reaction inhibitor. 3-MDG was synthesized by the inventors according to an already described process (Imaz, A.; Ayerbe, M.; Ramos, J.; Forcada, J. J. Polym. Sci. Part A: Polym. Chem. 2006, 44, 443-457).

### 2. Synthesis of poly(VCL-co-BA) and poly(VCL-co-BA-co-3-MDG) microgels

These two families of microgels of the invention were synthesized in a discontinuous reactor by means of an emulsion copolymerization process. In the synthesis of the first family [poly(VCL-co-BA) microgels], VCL was used as the main monomer and BA as the crosslinking agent, whereas in the synthesis of the second family [poly(VCL-co-BA-co-3-MDG) microgels], the monomer based on a carbohydrate, 3-O-methacryloyl-1,2:5,6-di-O-isopropylidene-D-glucofuranose (3-MDG) together with VCL (main monomer) and BA (crosslinking agent) was used. SDS was used as emulsifier and potassium persulfate as initiator. The buffer solution of sodium bicarbonate in water was added in the reaction to control the pH of the reaction mixture.

The particles of poly(VCL-co-BA) microgel were prepared using different concentrations of initiator at different reaction temperatures. Table 1 shows the recipes and reaction conditions used in the production of the particles of microgel crosslinked with BA [poly(VCL-co-BA)].

**Table 1**

| **Recipes and reaction conditions used in the production of the family of poly(VCL-co-BA) microgels** | | | | | | |
|---|---|---|---|---|---|---|
| Reaction | VCL (wt %) | BA (wt % M) | K₂S₂O₈ (wt % M) | NaHCO₃ (wt % M) | SDS (wt % M) | T (°C) |
| I1CL4BX | 1 | 4 | 1 | 0; 1 | 4 | 70 |
| I1CLX | 1 | 0; 4; 5; 7; 10 | 1 | 1 | 4 | 70 |
| IXCL4 | 1 | 4 | 0.3; 0.5; 1 | 0.3; 0.5; 1 | 4 | 70 |
| I1SX | 1 | 4 | 1 | 1 | 1; 2; 3; 4 | 70 |
| I1TX | 1 | 4 | 1 | 1 | 4 | 60; 70; 75; 80 |
| I1VCLX | 1; 3; 5 | 4 | 1 | 1 | 4 | 70 |
| Reaction conditions: rpm= 400; Reaction time= 5 hours | | | | | | |
| Variables: concentrations of VCL, BA, K₂S₂O₈, NaHCO₃ and SDS, and reaction temperature. | | | | | | |
| M = VCL | | | | | | |

| | | | | | | |
|---|---|---|---|---|---|---|
| The first column shows the reaction nomenclature [the number and/or the X followed by I indicates the amount of initiator (I, weight %) added with respect to the monomer (VCL), and the second number and/or X shows the amount of crosslinking agent (CL) and emulsifier (S). B means that the reaction is buffered and the value after B is the weight % of buffer with respect to VCL. | | | | | | |

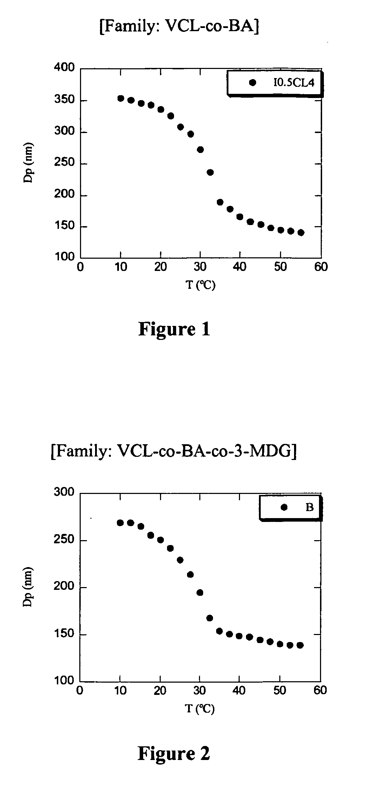

Figure 1 shows the average hydrodynamic diameter (Dp) vs. the temperature of the medium, of the particles of the microgel obtained in reaction I0.5CL4 [particular case of reaction IXCL4, in which the amount of initiator was 0.5 weight % with respect to VCL and the amount of buffer was 0.5 weight % with respect to VCL]. It is observed that, at low temperatures (below the volume phase transition temperature, VPTT), the microgels have a Dp of approximately 240 nm and that the particle size decreases when the temperature increases, clearly showing that at temperatures greater than the VPTT, the microgels deswell until approximately 115 nm.

In the synthesis of the poly(VCL-co-BA-co-3-MDG) microgels, different ratios of 3-MDG to VCL (13:87, 21:79, 30:70, 37:63) were used. The polymerization reactions were carried out at 70°C using 1 weight % of initiator and buffer with respect to the total amount of VCL and 3-MDG. Table 2 shows the recipe and the reaction conditions used in the production of the particles of poly(VCL-co-BA-co-3-MDG) microgel.

**Table 2**

| **Recipes and reaction conditions used in the production of the family of poly(VCL-co-BA-co-3-MDG) microgels** | |
|---|---|
| Reaction | 3-MDG/VCL |
| A | 13:87 |
| B | 21:79 |
| C | 30:70 |
| D | 37:63 |
| Reaction conditions: rpm = 400; Reaction time = 5 hours; Reaction temperature = 70°C. | |
| The concentrations of K₂S₂O₈ and NaHCO₃ are 1 weight % with respect to M (1 wt % M). | |
| The concentrations of BA and SDS are 4 wt % M. | |
| M = sum of VCL and 3-MDG comonomers (1 wt %). | |
| Variable: ratio between the VCL and 3-MDG comonomers. | |

Figure 2 shows the Dp vs. the temperature of the medium, of the particles of the microgel obtained in reaction B. It is observed that, at low temperatures (below the volume phase transition temperature, VPTT), the microgels have a Dp of approximately 270 nm and that the particle size decreases when the temperature increases, clearly showing that at temperatures greater than the VPTT, the microgels deswell until approximately 140 nm. It is furthermore observed that the incorporation of 3-MDG in the recipe causes a decrease in the swelling degree and in the VPTT.

### EXAMPLE 2

### Synthesis of microgels based on N-vinylcaprolactam and PEGDA [poly(VCL-co-PEGDA)]

### 1. Materials

The monomer, N-vinylcaprolactam (VCL, Aldrich); the initiator: potassium persulfate (K₂S₂O₈, Fluka); the buffer: sodium bicarbonate (NaHCO₃); the crosslinking agent: poly(ethylene glycol diacrylate) (PEGDA); the emulsifier: sodium dodecyl sulfate (SDS, Aldrich); the H¹-NMR standard, sodium acetate, and deuterium oxide (D₂O, Aldrich) were used as they were purchased. Twice-deionized water was used throughout the process. Hydroquinone (Fluka) was used as the polymerization reaction inhibitor.

### 2. Synthesis of poly(VCL-co-PEGDA) microgels

The particles of microgel were synthesized with different concentrations of crosslinking agent by means of an emulsion polymerization process in a discontinuous reactor. VCL was used as the main monomer and PEGDA as the crosslinking agent. The emulsifier used was SDS. Table 3 shows the recipe and the reaction conditions used in the production of poly(VCL-co-PEGDA).

**Table 3**

| **Recipes and reaction conditions used in the production of the family of microgels of poly(VCL-co-PEGDA)** | |
|---|---|
| Reaction | PEGDA (wt % M) |
| PE2 | 2 |
| PE4 | 4 |
| PE6 | 6 |
| PE8 | 8 |
| PE10 | 10 |
| Reaction conditions: rpm = 400; Reaction time = 5 hours; | |
| Reaction temperature = 70°C. | |
| The concentrations of K₂S₂O₈ and NaHCO₃ are 1 wt % M | |
| The concentration of SDS is 4 wt % M. | |
| M = VCL (1 wt %). | |
| Variable: concentration of PEGDA. | |

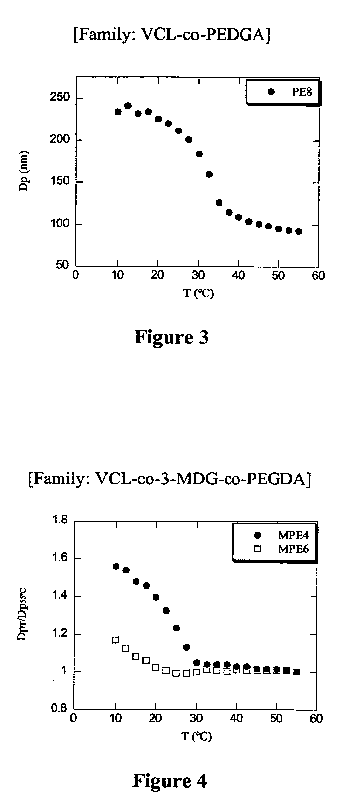

Figure 3 shows the Dp vs. the temperature of the medium, of the particles of the microgel obtained in reaction PE8. It is observed that, at low temperatures (below the volume phase transition temperature, VPTT), the microgels have a Dp of approximately 235 nm and that the particle size decreases when the temperature increases, clearly showing that at temperatures greater than the VPTT, the microgels deswell until approximately 95 nm.

### EXAMPLE 3

### Synthesis of microgels based on N-vinylcaprolactam and PEGDA [poly(VCL-co-3-MDG-co-PEGDA)

### 1. Materials

The monomer, N-vinylcaprolactam (VCL, Aldrich); the initiator: potassium persulfate (K₂S₂O₈, Fluka); the buffer: sodium bicarbonate (NaHCO₃); the crosslinking agent: poly(ethylene glycol diacrylate) (PEGDA); the emulsifier: sodium dodecyl sulfate (SDS, Aldrich); the H¹-NMR standard, sodium acetate, and deuterium oxide (D₂O, Aldrich) were used as they were purchased. 3-MDG was synthesized by the inventors according to an already described process (Imaz, A.; Ayerbe, M.; Ramos, J.; Forcada, J. J. Polym. Sci. Part A: Polym. Chem. 2006, 44, 443-457). Twice-deionized water was used throughout the process. Hydroquinone (Fluka) was used as the polymerization reaction inhibitor.

### 2. Synthesis of poly(VCL-co-3-MDG-co-PEGDA) microgels

The particles of microgel were synthesized with different concentrations of crosslinking agent by means of an emulsion polymerization process in a discontinuous reactor. 3-O-methacryloyl-1,2:5,6-di-O-isopropylidene-D-glucofuranose (3-MDG) was used as the monomer based on a carbohydrate together with VCL (main monomer) and PEGDA as the crosslinking agent. The emulsifier used was SDS. Table 4 shows the recipe and the reaction conditions used in the production of poly(VCL-co-3-MDG-co-PEGDA).

**Table 4**

| | |
|---|---|
| **Recipes and reaction conditions used in the production of the family of microgels of poly(VCL-co-3-MDG-co-PEGDA)** | |

| Reaction | PEGDA (wt % M) |
|---|---|
| MPE2 | 2 |
| MPE4 | 4 |
| MPE6 | 6 |
| Reaction conditions: rpm= 300; Reaction time = 5 hours; | |
| Reaction temperature = 70°C. | |
| The concentrations of K₂S₂O₈ and NaHCO₃ are 1 wt % M | |
| The concentration of SDS is 4 wt % M. | |
| M = sum of VCL and 3-MDG comonomers (1 wt %). | |
| VCL/3-MDG = 80/20 | |
| Variable: concentration of PEGDA. | |

Figure 4 shows the Dp_{T}/Dp_{55°C} vs. the temperature of the medium, of the particles of the microgels obtained in reactions MPE4 and MPE6, wherein it can be observed that when the concentration of crosslinking agent increases, the swelling ratio decreases.

### EXAMPLE 4

### Synthesis of microgels based on N-vinylcaprolactam and PEGDA [poly(VCL-co-3-MDG-co-PEGDA-co-AA)

### 1. Materials

The monomer, N-vinylcaprolactam (VCL, Aldrich); the initiator: potassium persulfate (K₂S₂O₈, Fluka); the buffer: sodium bicarbonate (NaHCO₃); then crosslinking agent: poly(ethylene glycol diacrylate) (PEGDA); the emulsifier: sodium dodecyl sulfate (SDS, Aldrich); the H¹-NMR standard, sodium acetate, and deuterium oxide (D₂O, Aldrich) were used as they were purchased. 3-MDG was synthesized by the inventors according to an already described process (Imaz, A.; Ayerbe, M.; Ramos, J.; Forcada, J. J. Polym. Sci. Part A: Polym. Chem. 2006, 44, 443-457). Acrylic acid (AA) (99%) was supplied by Fluka. Twice-deionized water was used throughout the process. Hydroquinone (Fluka) was used as the polymerization reaction inhibitor.

### 2. Synthesis of poly(VCL-co-3-MDG-co-PEGDA-co-AA) microgels

The particles of microgel were synthesized with different concentrations of acrylic comonomer by means of an emulsion polymerization process in a discontinuous reactor. 3-MDG was used as the monomer based on a carbohydrate together with VCL (main monomer) and PEGDA as the crosslinking agent. Acrylic acid (AA) was used as the acrylic comonomer. The emulsifier used was SDS. Table 5 shows the recipe and the reaction conditions used in the production of poly(VCL-co-3-MDG-co-PEGDA-co-AA).

**Table 5**

| **Recipes and reaction conditions used in the production of the family of microgels of poly(VCL-co-3-MDG-co-PEGDA-co-AA)** | | | |
|---|---|---|---|
| Reaction | AA (wt % M) | NaHCO₃ (wt % M) | SDS (wt % M) |
| AA2 | 2 | 6 | 4 |
| AA4 | 4 | 9 | 4 |
| AA6 | 6 | 8 | 1 |
| Reaction conditions: rpm= 250; Reaction time= 5 hours; | | | |
| Reaction temperature= 70°C. | | | |
| The concentration of K₂S₂O₈ is 1 wt % M; the concentration of PEGDA is 4 wt % M. | | | |
| M = sum of VCL and 3-MDG comonomers (1 wt %). | | | |
| VCL/3-MDG = 80/20 | | | |
| Variable: concentration of AA | | | |

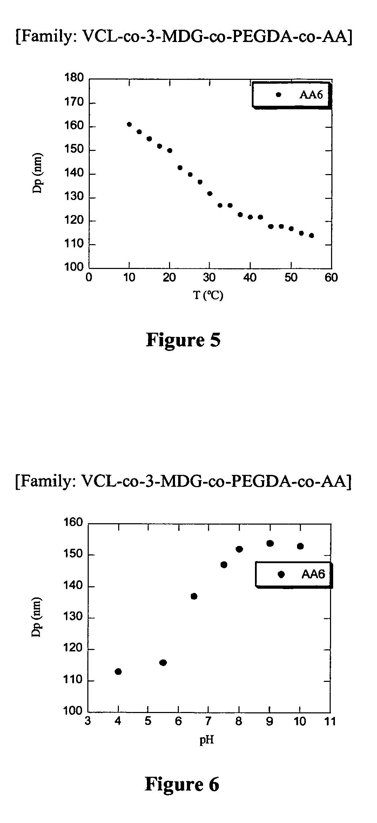

Figure 5 shows the Dp vs. the temperature of the medium, of the particles of the microgel obtained in reaction AA6. It is observed that, at low temperature (10°C), the microgels have a Dp of approximately 160 nm and that the particle size decreases when the temperature increases.

Figure 6 shows the Dp vs. the pH of the medium, of the particles of the microgel obtained in reaction AA6. It is observed that when the pH of the medium increases, the Dp of the microgels increases due to the repulsive interactions between the ionized carboxyl groups.

### EXAMPLE 5

### Absorption of calcein by the particles of microgels of the invention

This example was carried out to analyze the ability to uptake of a model molecule (calcein) by different microgels of the invention belonging to different families.

### 1. Materials and methods

The microgels of the invention used were the microgels identified as I1CL4B1 [microgel obtained in Example 1 (Table 1) belonging to the poly(VCL-co-BA) family], B [microgel obtained in Example 2 (Table 2) belonging to the poly(VCL-co-BA-co-3-MDG)family], PE6 [microgel obtained in Example 2 (Table 3) belonging to the poly(VCL-co-PEGDA) family], MPE4 [microgel obtained in Example 3 (Table 4) belonging to the poly(VCL-co-3-MDG-co-PEGDA) family] and AA6 [microgel obtained in Example 4 (Table 5) belonging to the poly(VCL-co-3-MDG-co-PEGDA-co-AA) family].

The model molecule selected for carrying out this example was calcein (Aldrich), the molecular formula of which is the following:

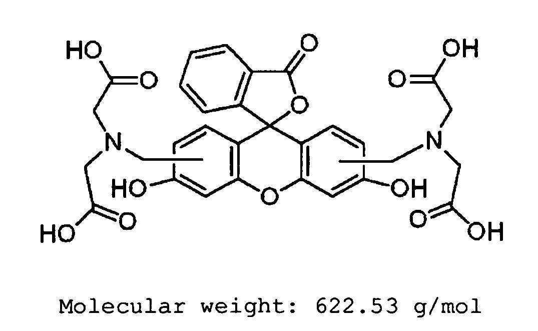

Twice-deionized water was also used.

### Analysis method

To determine the absorption of calcein, a calcein stock solution (200 ppm) at pH 3 was prepared on one hand for the purpose of determining in the spectrophotometer the optimal absorption wavelength (the maximum in the absorbance vs. wavelength curve); solutions with different concentrations of calcein were furthermore prepared to obtain the corresponding calibration curve.

On the other hand, different preparations with different concentrations of calcein [5-200 ppm (equivalent to approximately 0.008-0.32 mM)] at the same concentration of microgel (0.05 wt %), at pH 3, were prepared and were stirred for 60 hours by means of orbital stirring. The microgel was then separated from the aqueous medium by centrifugation at 20°C, 10,000 rpm, for 2 hours, using a Centrikon T-2149 ultracentrifuge (Kontron Instruments) and the concentration of calcein in equilibrium in the supernatant was analyzed by means of UV-spectrophotometry at 478 nm in a Spectronic Genesys 5 spectrophotometer. The resulting absorption values were compared with those obtained previously in the calibration curves, and the concentration of calcein in equilibrium was thus obtained.

### 2. Results

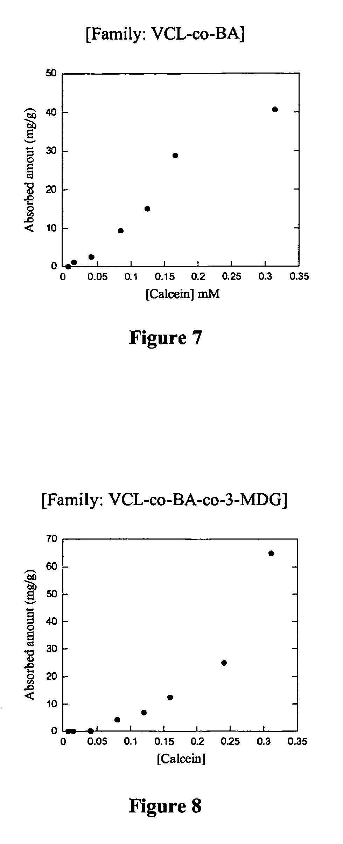

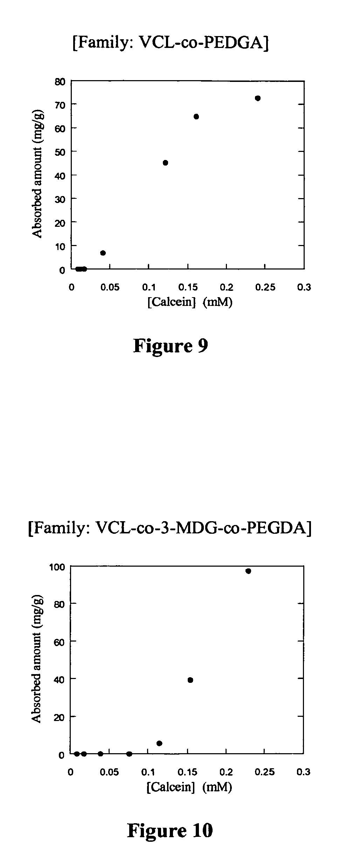

Calcein was loaded in the biocompatible microgels of the invention at pH 3 because at this pH the calcein carboxyl groups are protonated (-COOH). The results obtained are shown in Figures 5-11, wherein the amount of calcein absorbed (mg of calcein/g of microgel) by the different biocompatible microgels of the invention assayed can be observed against the concentration of calcein. Said results clearly show the potential of said assayed microgels as transport systems for biologically active agents; in particular, said microgels can absorb any biologically active agent containing -COOH groups.

### EXAMPLE 6

### Biocompatibility and toxicity assays of biocompatible microgels of the invention

This example was carried out to analyze the *in vitro* biocompatibility and toxicity of biocompatible microgels of the invention. Live cells were counted by means of fluorimetry for the purpose of finding out the limit value of the concentration of microgel that does not affect neuron viability.

### 1. Materials and methods

To carry out this example, calcein-AM (Invitrogen) [a fluorescent material which is incorporated by viable cells]; the microgel identified as I1CL4B1 [microgel obtained in Example 1 (Table 1) belonging to the poly(VCL-co-BA) family - particular case of reaction I1CL4BX, in which the amount of buffer is 1% by weight with respect to VCL]; 18-day embryonic rat primary neuron cultures with a high neuron content and twice-deionized water were used.

### 1.1 Preparation of the cell culture

Cortical lobe neurons of 18-day Sprague-Dawley rat embryos were cultured using previously described processes [Cheung et al., Neuropharmacology, 1998, 37:1419-1429; Larm et al., European Journal of Pharmacology 1996, 35:567-576]. The neurons were resuspended in Neurobasal B27 medium supplemented with 10% fetal calf serum (FCS) and were seeded on a poly-L-ornithine-coated glass support with a diameter of 12 mm at a concentration of 5x10⁴ cells per support. On the following day, the medium was changed for another serum-free medium (Neurobasal B27 Minus AO medium). The cultures were maintained in a humidified incubator (5% CO₂; 37°C) and were used 8-10 days after plating [Brewer et al., Journal of Neuroscience Research 1993, 35:567-576].

### 1.2 Cell viability analysis

The cell toxicity and viability assays were carried out using neuron cultures seeded at 5x10⁴ cells/support, as has already been described [Schubert & Piasecki, The Official Journal of the Society for Neuroscience, 2001, 21:7455-7462] with some modifications. The neurons were maintained in Neurobasal medium at 37°C and 5% CO₂ for 9 or 16 days. To evaluate the effect of the particles of the assayed microgel on cell viability, different concentrations of particles of said microgel (1% of solid content diluted 1:100, 1:300 and 1:1000) were added for 24 or 72 hours. The cell viability was assayed by loading the cells with 1 µM calcein-AM [viable cultured cells (in different maturation stages) incorporate calcein-AM] for 30 minutes and the fluorescence was measured using a Synergy-HT fluorimeter (Bio-Tek Instruments). The data is shown as percentage of live cells over the total number of cells with respect to the control. All the experiments were carried out in quadruplicate and the values provided herein are the average values of at least 3 independent experiments. The concentrations at which the assayed microgel does not alter cell viability can thus be established.

### 2. Results

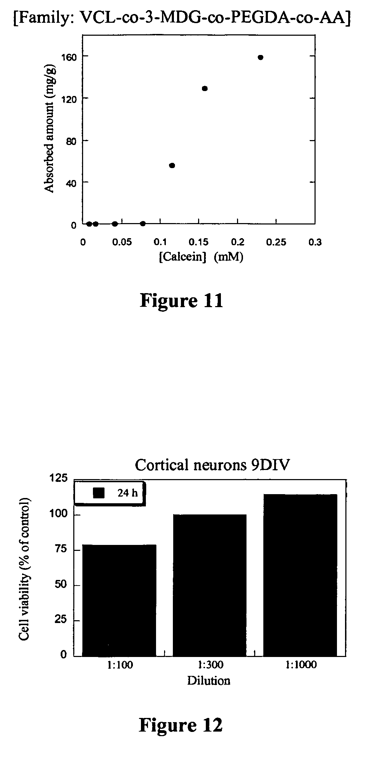

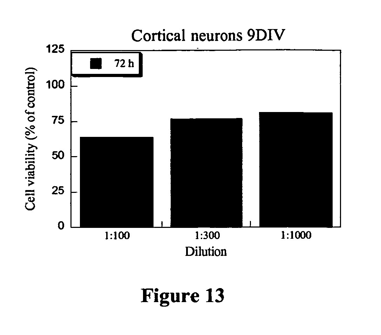

Figures 12 and 13 show the cell viabilities in 9-day embryonic rat cortical neuron *in vitro* cultures (9 DIV, "9 days *in vitro"*) after incubation for 24 hours (Figure 12) or for 72 hours (Figure 13) with the assayed biocompatible microgel of the invention [microgel identified as I1CL4B1, Table 1, belonging to the poly(VCL-co-BA) family]. The nanoparticles of said microgel showed low toxicity when the immature cells (9DIV) were incubated for 24 hours. However, the toxicity increased when the incubation time with the microgel was 72 hours.

When the effect of the concentration of the nanoparticles of the microgel on cell viability was analyzed, it was observed that in both cases, the 1:100 dilution (Figures 12 and 13, first column) showed the lowest cell viability. Likewise, it was observed that in the cells incubated for 24 hours, at 1:300 and 1:1000 dilutions (second and third columns on the right), the cell viability was 100%. Given that the cells did not die, the particles of the assayed microgel are completely biocompatible under the assayed conditions (low concentration of particles of microgel and an incubation period of 24 hours). These results allow concluding that said microgel, in the assayed conditions, can be used as a biocompatible device or system useful for biomedical applications.

The experiments carried out with cells incubated for 72 hours, at 1:300 and 1:1000 dilutions, clearly show that cell viability is higher than in the case of the 1:100 dilution, although in these conditions the assayed microgel was not completely biocompatible since the cell viability was 77% and 81% at 1:300 and 1:1000 dilutions, respectively.

## Claims

1. A biocompatible microgel comprising a polymeric network, said polymeric network comprising monomeric units interconnected with one another through a crosslinking agent, wherein said polymeric network can be obtained by polymerization in a dispersed medium of a biocompatible vinyl monomer, a comonomer based on a carbohydrate, and a crosslinking agent.

2. The microgel according to claim 1, wherein said biocompatible vinyl monomer is selected from N-vinylcaprolactam, N-vinylpyrrolidone and mixtures thereof.

3. The microgel according to claim 1, wherein said crosslinking agent is a difunctional monomer comprising at least two vinyl groups.

4. The microgel according to claim 3, wherein said crosslinking agent is selected from
a dextran with two or more vinyl groups;
N,N'-methylenebisacrylamide (BA)s
a compound of general formula 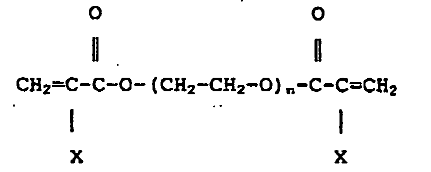 wherein
X is hydrogen or methyl, and
n is an integer comprised between 1 and 90; and mixtures thereof.

5. The microgel according to claim. 4, wherein said crosslinking agent is selected from ethylene glycol diacrylate (EGDA), poly(ethylene glycol diacrylate) (PEGDA), ethylene glycol dimethacrylate (EGDMA), poly(ethylene glycol dimethacrylate) (PEGDMA) and mixtures thereof.

6. The microgel according to claim 1, wherein said comonomer based on a carbohydrate comprises an acrylic or methacrylic group and a group derived from a glucoside, said group derived from a glucoside comprising a glucofuran and two isopropylidene groups.

7. The microgel according to claim 6, wherein said comonomer based on a carbohydrate is 3-O-methacryloyl-1,2:5,6-di-O-isopropylidene-D-glucofuranose (3-MOG).

8. The microgel according to claim 1, wherein said polymeric network further comprises a (meth)acrylic comonomer.

9. The microgel according to claim 8, wherein said acrylic comonomer is selected from acrylic acid, methacrylic acid and the pH-sensitive derivatives thereof, and mixtures thereof.

10. The microgel according to any of claims 1 to 9, further comprising a biologically active agent.

11. The microgel according to any of claims 1 to 10, in lyophilized form.

12. A pharmaceutical composition comprising a biocompatible microgels according to any of claims 10 or 11, and a pharmaceutically acceptable excipient or carrier.

13. A process for obtaining a biocompatible microgel according to claim 1, comprising the polymerization in a dispersed medium of a composition comprising a biocompatible vinyl monomer, a comonomer based on a carbohydrate, and a crosslinking agent.

14. The process according to claim 13, wherein said composition further comprises an acrylic comonomer.

15. The process according to claim 13 or 14, wherein said polymerization is carried out in the presence of a biologically active agent.

16. The process according to claim 13 or 14, further comprising mixing said biocompatible microgel with a solution comprising a biologically active agent.

## Patentansprüche

1. Ein biokompatibles Mikrogel, umfassend ein polymeres Netzwerk, wobei das polymere Netzwerk Monomereinheiten umfasst, die über ein Vernetzungsmittel miteinander verbunden sind, wobei das polymere Netzwerk durch Polymerisation eines biokompatiblen Vinylmonomers, eines Comonomers auf Basis eines Kohlenhydrats und eines Vernetzungsmittels in einem dispergierten Medium erhalten werden kann.

2. Das Mikrogel nach Anspruch 1, wobei das biokompatible Vinylmonomer aus N-Vinylcaprolactam, N-Vinylpyrrolidon und Gemischen davon ausgewählt ist.

3. Das Mikrogel nach Anspruch 1, wobei das Vernetzungsmittel ein difunktionelles Monomer ist, das mindestens zwei Vinylgruppen umfasst.

4. Das Mikrogel nach Anspruch 3, wobei das Vernetzungsmittel aus einem Dextran mit zwei oder mehr Vinylgruppen;
N,N'-Methylenbisacrylamid (BA);
einer Verbindung der allgemeinen Formel 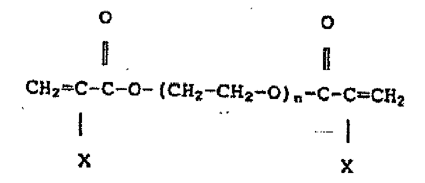 wobei
X Wasserstoff oder Methyl ist und
n eine ganze Zahl zwischen 1 und 90 ist;
und Gemischen davon ausgewählt ist.

5. Das Mikrogel nach Anspruch 4, wobei das Vernetzungsmittel aus Ethylenglykoldiacrylat (EGDA), Poly(ethylenglykoldiacrylat) (PEGDA), Ethylenglykoldimethacrylat (EGDMA), Poly(ethylenglykoldimethacrylat) (PEGDMA) und Gemischen davon ausgewählt ist.

6. Das Mikrogel nach Anspruch 1, wobei das Comonomer auf Basis eines Kohlenhydrats einen Acryl- oder Methacrylrest und einen von einem Glucosid abgeleiteten Rest umfasst, wobei der von einem Glucosid abgeleitete Rest ein Glucofuran und zwei Isopropylidenreste umfasst.

7. Das Mikrogel nach Anspruch 6, wobei das Comonomer auf Basis eines Kohlenhydrats 3-O-Methacryloyl-1,2:5,6-di-O-isopropyliden-D-glucofuranose (3-MDG) ist.

8. Das Mikrogel nach Anspruch 1, wobei das polymere Netzwerk weiter ein (Meth)acryl-Comonomer umfasst.

9. Das Mikrogel nach Anspruch 8, wobei das Acryl-Comonomer aus Acrylsäure, Methacrylsäure und den pH-empfindlichen Derivaten davon und Gemischen davon ausgewählt ist.

10. Das Mikrogel nach einem der Ansprüche 1 bis 9, weiter umfassend einen biologischen Wirkstoff.

11. Das Mikrogel nach einem der Ansprüche 1 bis 10 in lyophilisierter Form.

12. Ein Arzneimittel, umfassend ein biokompatibles Mikrogel nach einem der Ansprüche 10 oder 11 und einen pharmazeutisch verträglichen Exzipienten oder Träger.

13. Ein Verfahren zum Erhalt eines biokompatiblen Mikrogels nach Anspruch 1, umfassend die Polymerisation einer Zusammensetzung, die ein biokompatibles Vinylmonomer, ein Comonomer auf Basis eines Kohlenhydrats und ein Vernetzungsmittel umfasst, in einem dispergierten Medium.

14. Das Verfahren nach Anspruch 13, wobei die Zusammensetzung weiter ein Acryl-Comonomer umfasst.

15. Das Verfahren nach Anspruch 13 oder 14, wobei die Polymerisation in Gegenwart eines biologischen Wirkstoffes durchgeführt wird.

16. Das Verfahren nach Anspruch 13 oder 14, weiter umfassend Mischen des biokompatiblen Mikrogels mit einer einen biologischen Wirkstoff umfassenden Lösung.

## Revendications

1. Microgel biocompatible comprenant un réseau polymérique, ledit réseau polymérique comprenant des unités monomériques interconnectées les unes avec les autres par le biais d'un agent de réticulation, dans lequel ledit réseau polymérique peut être obtenu par polymérisation dans un milieu dispersé d'un monomère de vinyle biocompatible, d'un comonomère à base d'un glucide et d'un agent de réticulation.

2. Microgel selon la revendication 1, dans lequel ledit monomère de vinyle biocompatible est sélectionné parmi le N-vinylcaprolactame, la N-vinylpyrrolidone et des mélanges de ceux-ci.

3. Microgel selon la revendication 1, dans lequel ledit agent de réticulation est un monomère difonctionnel comprenant au moins deux groupes vinyle.

4. Microgel selon la revendication 3, dans lequel ledit agent de réticulation est sélectionné parmi
un dextrane avec deux groupes vinyle ou plus ;
le N,N'méthylènebisacrylamide (BA) ;
un composé de formule générale 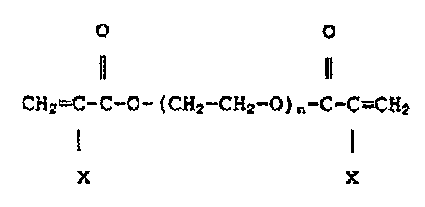 dans laquelle
X est hydrogène ou méthyle, et
n est un nombre entier compris entre 1 et 90 ; et des mélanges de ceux-ci.

5. Microgel selon la revendication 4, dans lequel ledit agent de réticulation est sélectionné parmi le diacrylate d'éthylène glycol (EGDA), le diacrylate de polyéthylène glycol (PEGDA), le diméthacrylate d'éthylène glycol (EGDMA), le diméthacrylate de polyéthylène glycol (PEGDMA) et des mélanges de ceux-ci.

6. Microgel selon la revendication 1, dans lequel ledit comonomère à base d'un glucide comprend un groupe acrylique ou méthacrylique et un groupe dérivé d'un glucoside, ledit groupe dérivé d'un glucoside comprenant un glucofurane et deux groupes isopropylidène.

7. Microgel selon la revendication 6, dans lequel ledit comonomère à base d'un glucide est le 3-O-méthacryloyl-1,2:5,6-di-O-isopropylidène-D-glucofuranose (3-MDG).

8. Microgel selon la revendication 1, dans lequel ledit réseau polymérique comprend en outre un comonomère (méth)acrylique.

9. Microgel selon la revendication 8, dans lequel ledit comonomère acrylique est sélectionné parmi l'acide acrylique, l'acide méthacrylique et les dérivés sensibles au pH de ceux-ci, et des mélanges de ceux-ci.

10. Microgel selon l'une quelconque des revendications 1 à 9, comprenant en outre un agent biologiquement actif.

11. Microgel selon l'une quelconque des revendications 1 à 10, sous forme lyophilisée.

12. Composition pharmaceutique comprenant un microgel biocompatible selon l'une quelconque des revendications 10 ou 11, et excipient ou vecteur pharmaceutiquement acceptable.

13. Procédé d'obtention d'un microgel biocompatible selon la revendication 1, comprenant la polymérisation dans un milieu dispersé d'une composition comprenant un monomère de vinyle biocompatible, un comonomère à base d'un glucide et un agent de réticulation.

14. Procédé selon la revendication 13, dans lequel ladite composition comprend en outre un comonomère acrylique.

15. Procédé selon la revendication 13 ou 14, dans lequel ladite polymérisation est réalisée en présence d'un agent biologiquement actif.

16. Procédé selon la revendication 13 ou 14, comprenant en outre le mélange dudit microgel biocompatible avec une solution comprenant un agent biologiquement actif.
